# EUROPEAN PATENT APPLICATION

(11) **EP 2 047 848 A1**
(43) Date of publication of application: **15.04.2009**
(21) Application number: 07768185.6
(22) Date of filing: 05.07.2007
(51) Int. Cl.: A61K 31/195, A61K 31/197, A61K 31/221, A61K 38/00, A61P 21/04, A61P 43/00, C12N 15/09

(54) **METHOD OF TREATING GENETIC DISEASE CAUSED BY NONSENSE MUTATION**

(30) Priority: 05.07.2006 US 806580 P
(71) Applicant: The University of Tokyo, Bunkyo-Ku, Tokyo 113-8654 (JP); University of Maryland, Baltimore, Baltimore MD 21201 (US)
(72) Inventor: MATSUDA, Ryoichi, Bunkyo-Ku Tokyo 113-8654 (JP); SHIOZUKA, Masataka, Bunkyo-Ku Tokyo 113-8654 (JP); MACKERELL, JR., Alexander, 21230-2130 Maryland (US)
(74) Representative: Krauss, Jan
(86) International application number: PCT/JP2007/063436
(87) International publication number: WO 2008/004610

(57) **Abstract**

An object of the present invention is to provide compounds having read-through activity for use in treatment methods of genetic diseases caused by nonsense mutation, to provide pharmaceutical compositions comprising the compound, and to provide a treatment method of genetic diseases caused by nonsense mutation comprising administering the compound.

The present invention can provide a method of producing wild type normal protein in a living body of a mammal from a gene with a premature termination codon being generated by a mutation, wherein the method comprises administering a compound expressed by the following formula (VI): 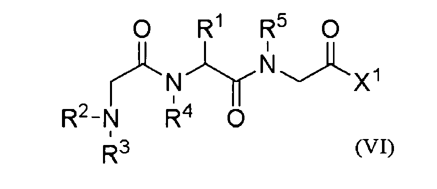 (wherein R¹, R², R³, R⁴, R⁵ and X¹ in the formula are as defined in description) or the like to the mammal.

## Description

### TECHNICAL FIELD

The present invention relates to treatment methods and pharmaceutical compositions used in the treatment methods for genetic diseases caused by nonsense mutation, such as muscular dystrophy, Duchenne muscular dystrophy, multiple sclerosis, infantile neuronal ceroid lipofuscinosis, Alzheimer's disease, Tay-Sachs disease, neural tissue degeneration, Parkinson's disease, chronic rheumatoid arthritis, graft-versus-host disease, arthritis, hemophilia, von Willebrand disease, ataxia telangiectasia, thalassemia, nephrolithiasis, osteogenesis imperfecta, cirrhosis, neurofibroma, bullous disease, lysosomal storage disease, Hurler's disease, familial cholesterolemia, cerebellar ataxia, tuberous sclerosis, familial erythrocytosis, immune deficiency, kidney disease, lung disease, cystic fibrosis, familial hypercholesterolemia, pigmentary retinopathy, amyloidosis, atheroscrerosis, gigantism, dwarfism, hypothyroidism, hyperthyroidism, aging, obesity, diabetes mellitus, Niemann-Pick disease, Marfan syndrome, and cancer.

### BACKGROUND ART

A genetic disease caused by nonsense mutation is attributed to a generated premature termination codon due to a point mutation in a gene which results in inhibition of protein expression. For example, Duchenne muscular dystrophy is attributed to absence of dystrophin protein in Sarcolemma. The disease has a generated premature termination codon due to a mutation in a dystrophin gene located on X-chromosome which brings interruption and termination of translation at the mutation site, resulting in inhibition of expression of dystrophin related proteins and consequently in absence of dystrophin protein.

Genetic diseases caused by nonsense mutation may be treated by a treatment method comprising introducing genes exogenously, a so called gene therapy. However, the treatment method has problems in safety of virus vectors and low gene transfer efficiency of alternative vectors. Therefore, it is hard for the present to actually use the treatment method.

Meanwhile, it has been found that when a particular compound was administered to a patient who has a deficiency in specific protein due to a nonsense mutation leading to a premature termination codon, the compound effects on ribosomes and brings ribosomes to read through the termination codon during translation (hereinafter, may be abbreviated to read-through), which results in a synthesis of wild type normal protein. Gentamicin, an aminoglycoside antibiotic, has been known to be a compound having such read-through activity. Politano et al. has been found that administration of gentamicin to patients with Duchenne muscular dystrophy led to accumulation of dystrophin protein (Non-patent document 1). It has been also reported that topical administration of gentamicin to airway epithelium of patients with cystic fibrosis could correct typical electrophysiological abnormalities of the patients (Non-patent document 2). Further, administration of gentamicin is being studied in clinical trials in the United States for treatment of genetic diseases. In addition, negamycin, a dipeptide antibiotic, has been also reported to have read-through activity, and administration thereof has been found to restore a dystrophin expression in mouse models for muscular dystrophy (Patent document 1).

Patent Document 1: International publication No. WO2002/102361 pamphlet
Non-patent Document 1: Acta.Myol.Vol, 22, p. 15-21, 2003
Non-patent Document 2: N.Engl.J.Med., Vol. 349, p. 1433-1441, 2003

### DISCLOSURE OF THE INVENTION

### [PROBLEM TO BE SOLVED BY THE INVENTION]

An object of the present invention is to provide compounds having read-through activity for use in treatment methods of genetic diseases caused by nonsense mutation, to provide pharmaceutical compositions comprising the compound, and to provide a treatment method of genetic diseases caused by nonsense mutation comprising administering the compound.

### [MEANS FOR SOLVING THE OBJECT]

The present inventors have conducted concentrated studies to meet the aforementioned object and have discovered compounds having read-through activity, and thus achieved the present invention.

That is to say, the present invention relates to a method of producing wild type normal protein from a gene with a premature termination codon being generated by a mutation, wherein the method comprises conducting ribosomal translation in the presence of a compound expressed by any one of the following formulas (I) to (V) or a pharmacologically acceptable salt thereof: 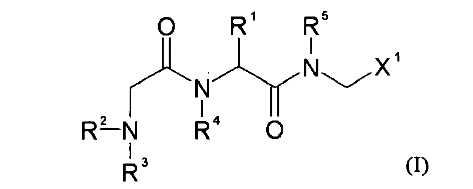 wherein, in Formula (I), R¹ represents C₁₋₁₀ alkyl; R² and R³ are independently selected from hydrogen atom and C₁₋₁₀ alkyl which may have a substituent; R⁴ and R⁵ are independently selected from hydrogen atom and C₁₋₁₀ alkyl; X¹ represents carboxy, lower alkoxycarbonyl which may have a substituent, carbamoyl which may have a substituent, or hydrazinocarbonyl which may have a substituent, 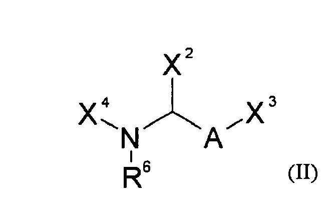 wherein, in Formula (II), X², X³ and X⁴ independently represent carboxy, alkoxycarbonyl which may have a substituent, carbamoyl which may have a substituent, or hydrazinocarbonyl which may have a substituent; R⁶ represents hydrogen atom or C₁₋₁₀ alkyl which may have a substituent; A represents alkylene which may have a substituent, 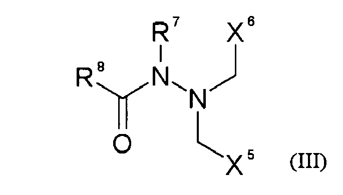 wherein X⁵ and X⁶ are independently selected from carboxy, alkoxycarbonyl which may have a substituent, and carbamoyl which may have a substituent; R⁷ represents hydrogen atom or C₁₋₁₀ alkyl which may have a substituent; R⁸ represents amino which may have a substituent, C₁₋₁₀ alkyl which may have a substituent, or C₁₋₁₀ alkenyl which may have a substituent, 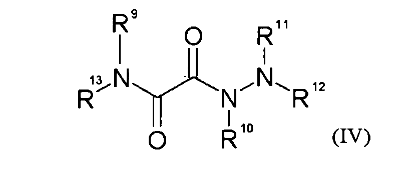 wherein R⁹, R¹⁰ and R¹³ independently represent hydrogen atom or C₁₋₁₀ alkyl which may have a substituent; R¹¹ and R¹² independently represent hydrogen atom, C₁₋₁₀ alkyl which may have a substituent, or carbonyl which is substituted with an aromatic substituent, or R¹¹ and R¹² may mutually bonded to form double bond leading to =CR¹⁷R¹⁸, wherein R¹⁷ and R¹⁸ are independently selected from hydrogen atom, C₁₋₁₀ alkyl, and carbamoylalkyl which may have a substituent, 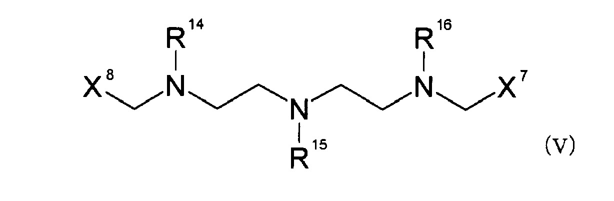 wherein X⁷ and X⁸ are independently selected from carboxy, alkoxycarbonyl which may have a substituent, and carbamoyl which may have a substituent; R¹⁴, R¹⁵ and R¹⁶ independently represent hydrogen atom or C₁₋₁₀ alkyl which may have a substituent.

The present invention also relates to the aforementioned method of producing wild type normal protein, wherein the method comprises administering the compound to a mammal, and wild type normal protein is produced in a living body of the mammal.

Further, the present invention relates to the aforementioned method of producing wild type normal protein, wherein the method comprises administering the compound to a patient with a genetic disease caused by nonsense mutation.

Furthermore, the present invention relates to the aforementioned method of producing wild type normal protein, wherein the method comprises administering the compound to a patient with a disease selected from muscular dystrophy, Duchenne muscular dystrophy, multiple sclerosis, infantile neuronal ceroid lipofuscinosis, Alzheimer's disease, Tay-Sachs disease, neural tissue degeneration, Parkinson's disease, chronic rheumatoid arthritis, graft-versus-host disease, arthritis, hemophilia, von Willebrand disease, ataxia telangiectasia, thalassemia, nephrolithiasis, osteogenesis imperfecta, cirrhosis, neurofibroma, bullous disease, lysosomal storage disease, Hurler's disease, familial cholesterolemia, cerebellar ataxia, tuberous sclerosis, familial erythrocytosis, immune deficiency, kidney disease, lung disease, cystic fibrosis, familial hypercholesterolemia, pigmentary retinopathy, amyloidosis, atheroscrerosis, gigantism, dwarfism, hypothyroidism, hyperthyroidism, aging, obesity, diabetes mellitus, Niemann-Pick disease, Marfan syndrome, and cancer.

Further, the present invention relates to the aforementioned method of producing wild type normal protein, wherein the method comprises administering the compound to a patient with muscular dystrophy.

Furthermore, the present invention relates to a pharmaceutical composition for use in treatment or prevention of a genetic disease caused by nonsense mutation, wherein the pharmaceutical composition comprises as an effective ingredient a compound expressed by any one of the aforementioned formulas (I) to (V) or a pharmacologically acceptable salt thereof.

Further, the present invention relates to the aforementioned pharmaceutical composition for use in treatment or prevention of a disease, wherein the disease is selected from a group consisting of muscular dystrophy, Duchenne muscular dystrophy, multiple sclerosis, infantile neuronal ceroid lipofuscinosis, Alzheimer's disease, Tay-Sachs disease, neural tissue degeneration, Parkinson's disease, chronic rheumatoid arthritis, graft-versus-host disease, arthritis, hemophilia, von Willebrand disease, ataxia telangiectasia, thalassemia, nephrolithiasis, osteogenesis imperfecta, cirrhosis, neurofibroma, bullous disease, lysosomal storage disease, Hurler's disease, familial cholesterolemia, cerebellar ataxia, tuberous sclerosis, familial erythrocytosis, immune deficiency, kidney disease, lung disease, cystic fibrosis, familial hypercholesterolemia, pigmentary retinopathy, amyloidosis, atheroscrerosis, gigantism, dwarfism, hypothyroidism, hyperthyroidism, aging, obesity, diabetes mellitus, Niemann-Pick disease, Marfan syndrome, and cancer.

Furthermore, the present invention relates to the aforementioned pharmaceutical composition for use in treatment or prevention of a disease, wherein the disease is muscular dystrophy.

Further, the present invention relates to a method of assay for a compound having read-through activity, wherein the method comprises:
a) a step of preparing host cells or transgenic animals which are transfected with a vector comprising the following: i) promoter, ii) a first translation start codon and a first reporter gene located downstream of the promoter, iii) a second translation start codon and a second reporter gene located downstream of the first reporter gene, iv) a sequence being located between the first reporter gene and the second translation start codon, and comprising a premature termination codon derived from a responsible gene of a genetic disease caused by nonsense mutation, and v) a translation termination codon located downstream of the second reporter gene;
b) a step of administering a test compound to the host cells or the transgenic animals; and
c) a step of comparing a ratio of an expression amount of the second reporter gene to that of the first reporter gene between in the presence of the test compound and in the absence thereof.

Furthermore, the present invention relates to an agent for read-through of a premature termination codon generated by a nonsense mutation, wherein the agent consists of a compound expressed by any one of the aforementioned formulas (I) to (V), a hydrate thereof, a solvate thereof, or a salt of any of the foregoing.

Further, the present invention relates to a method of producing wild type normal protein from a gene with a premature termination codon being generated by a mutation, wherein the method comprises conducting ribosomal translation in the presence of a compound expressed by any one of the following formulas (VI) to (X) or a pharmacologically acceptable salt thereof: 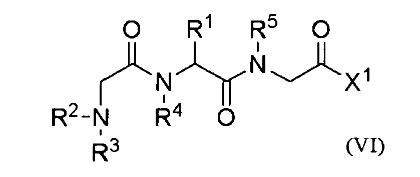 wherein, in the formula, R¹ represents C₁₋₁₀ alkyl; R² and R³ are independently selected from hydrogen atom, C₁₋₁₀ alkyl, and C₁₋₁₀ alkylcarbonyl; R⁴ and R⁵ are independently selected from hydrogen atom and C₁₋₁₀ alkyl; X¹ represents hydroxy, amino, C₁₋₁₀ alkoxy, C₁₋₁₀ alkylamino or di(C₁₋₁₀ alkyl)amino, 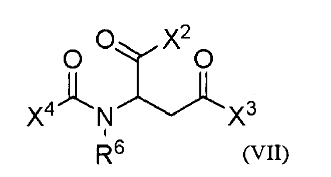 wherein, in the formula, X², X³ and X⁴ are independently selected from hydroxy, amino, C₁₋₁₀ alkoxy, C₁₋₁₀ alkylamino, and di(C₁₋₁₀ alkyl)amino; R⁶ represents hydrogen atom or C₁₋₁₀ alkyl, 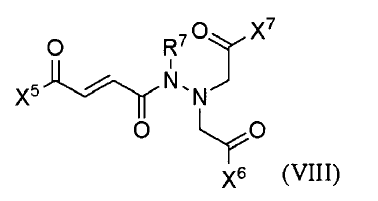 wherein X⁵, X⁶ and X⁷ are independently selected from hydroxy, amino, C₁₋₁₀ alkoxy, C₁₋₁₀ alkylamino, and di(C₁₋₁₀ alkyl)amino; R⁷ represents hydrogen atom or C₁₋₁₀ alkyl, 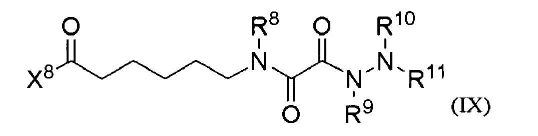 wherein X⁸ represents hydroxy, amino, C₁₋₁₀ alkoxy, C₁₋₁₀ alkylamino, and di(C₁₋₁₀ alkyl)amino; R⁸ and R⁹ independently represent hydrogen atom or C₁₋₁₀ alkyl; R¹⁰ and R¹¹ are independently selected from hydrogen atom, C₁₋₁₀ alkyl and C₁₋₁₀ alkylcarbonyl, 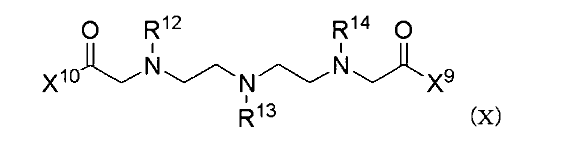 wherein X⁹ and X ¹⁰ are independently selected from hydroxy, amino, C₁₋₁₀ alkoxy, C₁₋₁₀ alkylamino, and di(C₁₋₁₀ alkyl)amino; R¹², R¹³ and R¹⁴ are independently selected from hydrogen atom, C₁₋₁₀ alkyl, C₁₋₁₀ alkylcarbonyl and -CH₂COX¹¹; X¹¹ represents hydroxyl, amino, C₁₋₁₀ alkoxy, C₁₋₁₀ alkylamino, and di(C₁₋₁₀ alkyl)amino.

Furthermore, the present invention relates to a pharmaceutical composition for use in treatment or prevention of genetic diseases caused by nonsense mutation, wherein the pharmaceutical composition comprises as an effective ingredient a compound expressed by any one of the aforementioned formulas (VI) to (X) or a pharmacologically acceptable salt thereof.

### [ADVANTAGE OF THE INVENTION]

The present invention provides compounds that cause read-through of a premature termination codon generated by a mutation, a read-through agent consisting of the compound, and a pharmaceutical composition comprising the compound. Further, the compounds, the read-through agent and the pharmaceutical composition can be used for production of a wild type normal protein from a gene in which the premature termination codon was generated, and thus, allow to conduct treatment or prevention of diseases attributable to a nonsense mutation that generates a premature termination codon.

### BRIEF DESCRIPTION OF THE DRAWINGS

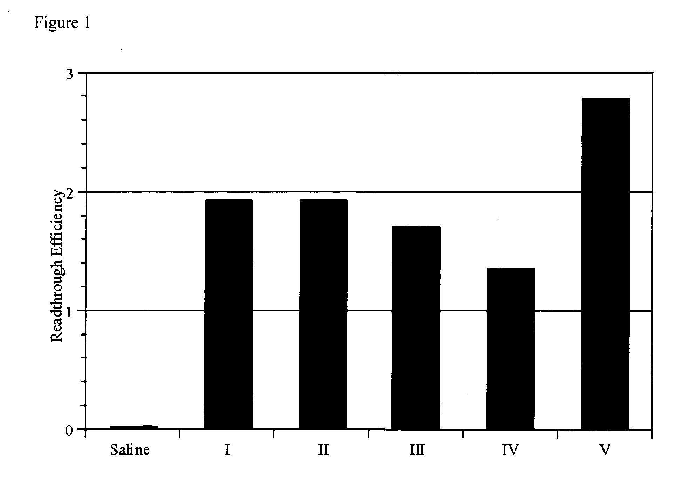

Figure 1 illustrates that all of compounds 1 to 5 showed a significant read-through activity as compared to saline control. In the figure, the compounds I to 5 are shown as I, II, III, IV, and V, respectively, and read-through activity is exhibited as Readthrough Efficiency. (Example 1)

### DETAILED DESCRIPTION OF THE INVENTION

In one aspect of the present invention, a method of producing wild type normal protein from a gene with a premature termination codon being generated by a mutation is provided, wherein the method comprises conducting ribosomal translation in the presence of a compound expressed by any one of the following formulas (I) to (V) or a pharmacologically acceptable salt thereof: 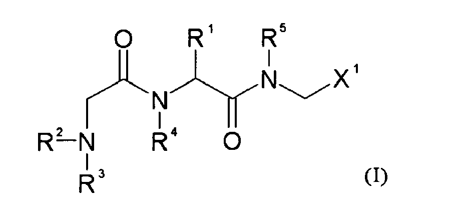 wherein, in the formula, R¹ represents C₁₋₁₀ alkyl; R² and R³ are independently selected from hydrogen atom and C₁₋₁₀ alkyl which may have a substituent; R⁴ and R⁵ are independently selected from hydrogen atom and C₁₋₁₀ alkyl; X¹ represents carboxy, lower alkoxycarbonyl which may have a substituent, carbamoyl which may have a substituent, or hydrazinocarbonyl which may have a substituent, 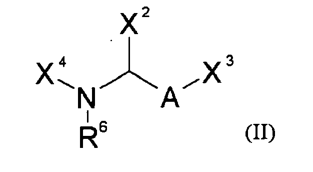 wherein, in the formula, X², X³ and X⁴ independently represent carboxy, alkoxycarbonyl which may have a substituent, carbamoyl which may have a substituent, or hydrazinocarbonyl which may have a substituent; R⁶ represents hydrogen atom or C₁₋₁₀ alkyl which may have a substituent; A represents alkylene which may have a substituent, 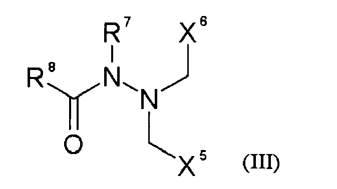 wherein X⁵ and X⁶ are independently selected from carboxy, alkoxycarbonyl which may have a substituent, and carbamoyl which may have a substituent; R⁷ represents hydrogen atom or C₁₋₁₀ alkyl which may have a substituent; R⁸ represents amino which may have a substituent, C₁₋₁₀ alkyl which may have a substituent, or C₁₋₁₀ alkenyl which may have a substituent, 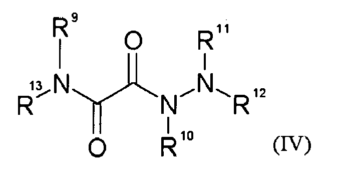 wherein R⁹, R¹⁰ and R¹³ independently represent hydrogen atom or C₁₋₁₀ alkyl which may have a substituent; R¹¹ and R¹² independently represent hydrogen atom, C₁₋₁₀ alkyl which may have a substituent, or carbonyl which is substituted with an aromatic substituent, or R¹¹ and R¹² may mutually bonded to form double bond leading to =CR¹⁷R¹⁸, wherein R¹⁷ and R¹⁸ are independently selected from hydrogen atom, C₁₋₁₀ alkyl, and carbamoylalkyl which may have a substituent, 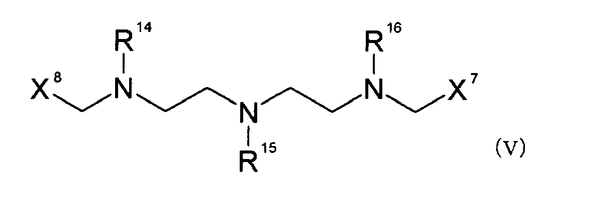 wherein X⁷ and X⁸ are independently selected from carboxy, alkoxycarbonyl which may have a substituent, and carbamoyl which may have a substituent; R¹⁴, R¹⁵ and R¹⁶ independently represent hydrogen atom or C₁₋₁₀ alkyl which may have a substituent.

In the aforementioned general formula (I), C₁₋₁₀ alkyl or C₁₋₁₀ alkyl which may have a substituent as represented by R¹ is preferably C₁₋₈ alkyl or C₁₋₈ alkyl which may have a substituent, more preferably C₁₋₆ alkyl or C₁₋₆ alkyl which may have a substituent, and further preferably C₁₋₄ alkyl or C₁₋₄ alkyl which may have a substituent. Preferable examples thereof include lower alkyl such as methyl, ethyl, propyl, and butyl, and particularly preferable examples include isopropyl and isobutyl.

C₁₋₁₀ alkyl which may have a substituent as represented by R² and R³ is preferably C₁₋₈ alkyl which may have a substituent, more preferably C₁₋₆ alkyl which may have a substituent, and further preferably C₁₋₄ alkyl which may have a substituent. Preferable examples thereof include lower-alkoxycarbonyl-lower-alkyl such as methoxycarbonylmethyl, methoxycarbonylethyl, methoxycarbonylpropyl, methoxycarbonylbutyl, ethoxycarbonylmethyl, ethoxycarbonylethyl, ethoxycarbonylpropyl, and ethoxycarbonylbutyl.

C₁₋₁₀ alkyl or C₁₋₁₀ alkyl which may have a substituent as represented by R⁴ and R⁵ is preferably C₁₋₈ alkyl or C₁₋₈ alkyl which may have a substituent, more preferably C₁₋₆ alkyl or C₁₋₆ alkyl which may have a substituent, and further preferably C₁₋₄ alkyl or C₁₋₄ alkyl which may have a substituent. Preferable examples thereof include lower alkyl such as methyl, ethyl, propyl, and butyl, and particularly preferable examples include isopropyl and isobutyl.

Alkoxycarbonyl which may have a substituent as represented by X¹ is preferably lower alkylcarbonyl such as methoxycarbonyl, ethoxycarbonyl, propylcarbonyl, and butoxycarbonyl. Carbamoyl which may have a substituent and hydrazinocarbonyl which may have a substituent may be monosubstituted, disubstituted, or all substituted, and examples of a substituent include lower alkyl such as methyl, ethyl, propyl, and butyl.

In the aforementioned general formula (II), alkoxycarbonyl which may have a substituent as represented by X², X³ and X⁴ is preferably exemplified by lower alkylcarbonyl such as methoxycarbonyl, ethoxycarbonyl, propylcarbonyl, and butoxycarbonyl. Carbamoyl which may have a substituent and hydrazinocarbonyl which may have a substituent may be monosubstituted, disubstituted, or all substituted, and substituents are exemplified by lower alkyl such as methyl, ethyl, propyl, and butyl.

C₁₋₁₀ alkyl which may have a substituent as represented by R⁶ is preferably C₁₋₈ alkyl which may have a substituent, more preferably C₁₋₆ alkyl which may have a substituent, and further preferably C₁₋₄ alkyl which may have a substituent. Preferable examples thereof include lower alkyl such as methyl, ethyl, propyl, and butyl. Alkylene which may have a substituent as represented by A is preferably exemplified by lower alkylene such as methylene, ethylene, and propylene.

In the aforementioned general formula (III), alkoxycarbonyl which may have a substituent as represented by X⁵ and X⁶ is preferably exemplified by methoxycarbonyl, ethoxycarbonyl, propylcarbonyl, and butoxycarbonyl. Carbamoyl which may have a substituent may be monosubstituted or disubstituted, and substituents are exemplified by lower alkyl such as methyl, ethyl, propyl, and butyl.

C₁₋₁₀ alkyl which may have a substituent as represented by R⁷ is preferably C₁₋₈ alkyl which may have a substituent, more preferably C₁₋₆ alkyl which may have a substituent, and further preferably C₁₋₄ alkyl which may have a substituent. Preferable examples thereof include lower alkyl such as methyl, ethyl, propyl, and butyl. Amino which may have a substituent as represented by R⁸ may have a substituent preferably exemplified by the following: lower alkyl such as methyl, ethylpropyl, and butyl; hydroxyl lower alkyl such as hydroxymethyl, hydroxyethy, hydroxypropyl, and hydroxybuthyl; and lower alkoxyalkyl such as methoxymethyl, methoxyethyl, methoxypropyl, and methoxybutyl. A substituent of C₁₋₁₀ alkyl which may have a substituent or of C₁₋₁₀ alkenyl which may have a substituent is preferably C₁₋₈ alkyl or C₁₋₈ alkenyl, which may have a substituent, more preferably C₁₋₆ alkyl or C₁₋₆ alkenyl, which may have a substituent, and further preferably C₁₋₄ alkyl or C₁₋₄ alkenyl, which may have a substituent. Preferable examples thereof include alkoxy such as hydroxy or methoxy, and ethoxy.

In the aforementioned general formula (IV), C₁₋₁₀ alkyl which may have a substituent as represented by R⁹, R¹⁰ and R¹³ is preferably C₁₋₈ alkyl which may have a substituent, more preferably C₁₋₆ alkyl which may have a substituent, and further preferably C₁₋₄ alkyl which may have a substituent. Preferable examples thereof include the following: lower alkyl such as methyl, ethylpropyl, and butyl; hydroxy lower alkyl such as hydroxymethyl, hydroxyethyl, hydroxypropyl, and hydroxybutyl; and lower alkoxyalkyl such as methoxymethyl, methoxyethyl, methoxypropyl, and methoxybutyl.

C₁₋₁₀ alkyl which may have a substituent as represented by R¹¹ and R¹² is preferably C₁₋₈ alkyl which may have a substituent, more preferably C₁₋₆ alkyl which may have a substituent, and further preferably C₁₋₄ alkyl which may have a substituent. Preferable examples thereof include the following: carboxy lower alkyl such as carboxymethyl, carboxyethyl, carboxypropyl, and carboxybutyl; and lower-alkoxy-lower-alkyl such as methoxycarbonylmethyl, methoxycarbonylethyl, methoxycarboxypropyl, and methoxycarbonylbutyl. Preferable examples of carbonyl substituted with an aromatic substituent include phenylcarbonyl and naphtylcarbonyl.

C₁₋₁₀ alkyl as represented by R¹⁷ and R¹⁸ is preferably C₁₋₈ alkyl, more preferably C₁₋₆ alkyl, and further preferably C₁₋₄ alkyl. Preferable examples thereof include lower alkyl such as methyl, ethyl, propyl, and butyl. Carbamoylalkyl which may have a substituent is preferably exemplified by the following: lower-alkylcarbamoyl-lower-alkyl such as methylcarbamoymethyl, ethylcarbamoylmethyl, propylcarbamoylmetyl, and butylcarbamoylmetyl; and cyclo(C₃₋₈)alkyl carbamoylmethyl such as cyclobutylcarbamoylmethyl, cyclopentylcarbamoylmethyl, and cyclohexylcarbamoylmethyl.

In the aforementioned general formula (V), alkoxycarbonyl which may have a substituent as represented by X⁷ and X⁸ is preferably exemplified by methoxycarbonyl, ethoxycarbonyl, propylcarbonyl, and butoxycarbonyl. Carbamoyl which may have a substituent may be monosubstituted or disubstituted, and substituents are exemplified by lower alkyl such as methyl, ethyl, propyl, and butyl.

C₁₋₁₀ alkyl which may have a substituent as represented by R¹⁴, R¹⁵ and R¹⁶ is preferably C₁₋₈ alkyl which may have a substituent, more preferably C₁₋₆ alkyl which may have a substituent, and further preferably C₁₋₄ alkyl which may have a substituent. Preferable examples thereof include the following: lower alkyl such as methyl, ethyl, propyl, and butyl; carboxy-lower-alkyl such as carboxymethyl, carboxymethyl, carboxypropyl, and carboxybutyl; lower-alkylcarbonyl-lower-alkyl; and carbamoyl-lower-alkyl which may have a substituent.

A compound preferably used in the aforementioned method may be represented by any one the following formula (VI) to (X): 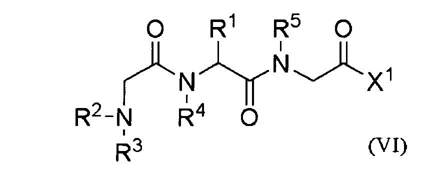 wherein, in the formula, R¹ represents C₁₋₁₀ alkyl; R² and R³ are independently selected from hydrogen atom, C₁₋₁₀ alkyl, and C₁₋₁₀ alkylcarbonyl; R⁴ and R⁵ are independently selected from hydrogen atom and C₁₋₁₀ alkyl; X¹ represents hydroxy, amino, C₁₋₁₀ alkoxy, C₁₋₁₀ alkylamino or di(C₁₋₁₀ alkyl)amino, 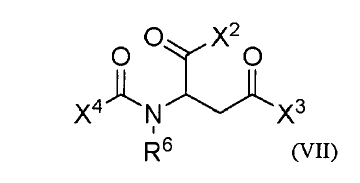 wherein, in the formula, X², X³ and X⁴ are independently selected from hydroxy, amino, C₁₋₁₀ alkoxy, C₁₋₁₀ alkylamino, and di(C₁₋₁₀ alkyl)amino; R⁶ represents hydrogen atom or C₁₋₁₀ alkyl, 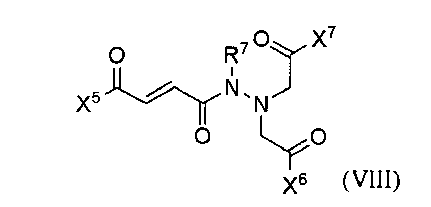 wherein X⁵, X⁶ and X⁷ are independently selected from hydroxy, amino, C₁₋₁₀ alkoxy, C₁₋₁₀ alkylamino, and di(C₁₋₁₀ alkyl)amino; R⁷ represents hydrogen atom or C₁₋₁₀ alkyl, 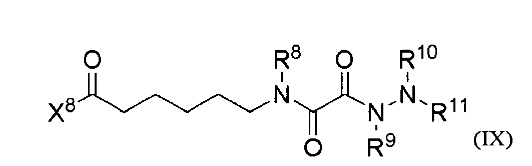 wherein X⁸ represents hydroxy, amino, C₁₋₁₀ alkoxy, C₁₋₁₀ alkylamino, and di(C₁₋₁₀ alkyl)amino; R⁸ and R⁹ independently represent hydrogen atom or C₁₋₁₀ alkyl; R¹⁰ and R¹¹ are independently selected from hydrogen atom, C₁₋₁₀ alkyl and C₁₋₁₀ alkylcarbonyl, 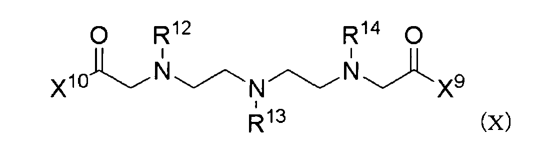 wherein X⁹ and X¹⁰ are independently selected from hydroxy, amino, C₁₋₁₀ alkoxy, C₁₋₁₀ alkylamino, and di(C₁₋₁₀ alkyl)amino; R¹² , R¹³ and R¹⁴ are independently selected from hydrogen atom, C₁₋₁₀ alkyl, C₁₋₁₀ alkylcarbonyl and -CH₂COX¹¹; X¹¹ represents hydroxyl, amino, C₁₋₁₀ alkoxy, C₁₋₁₀ alkylamino, and di(C₁₋₁₀ alkyl)amino.

A compound more preferably used in the aforementioned method may be specifically exemplified by compounds shown in the following table.

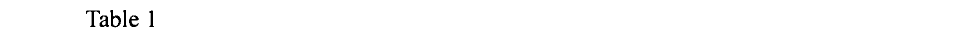

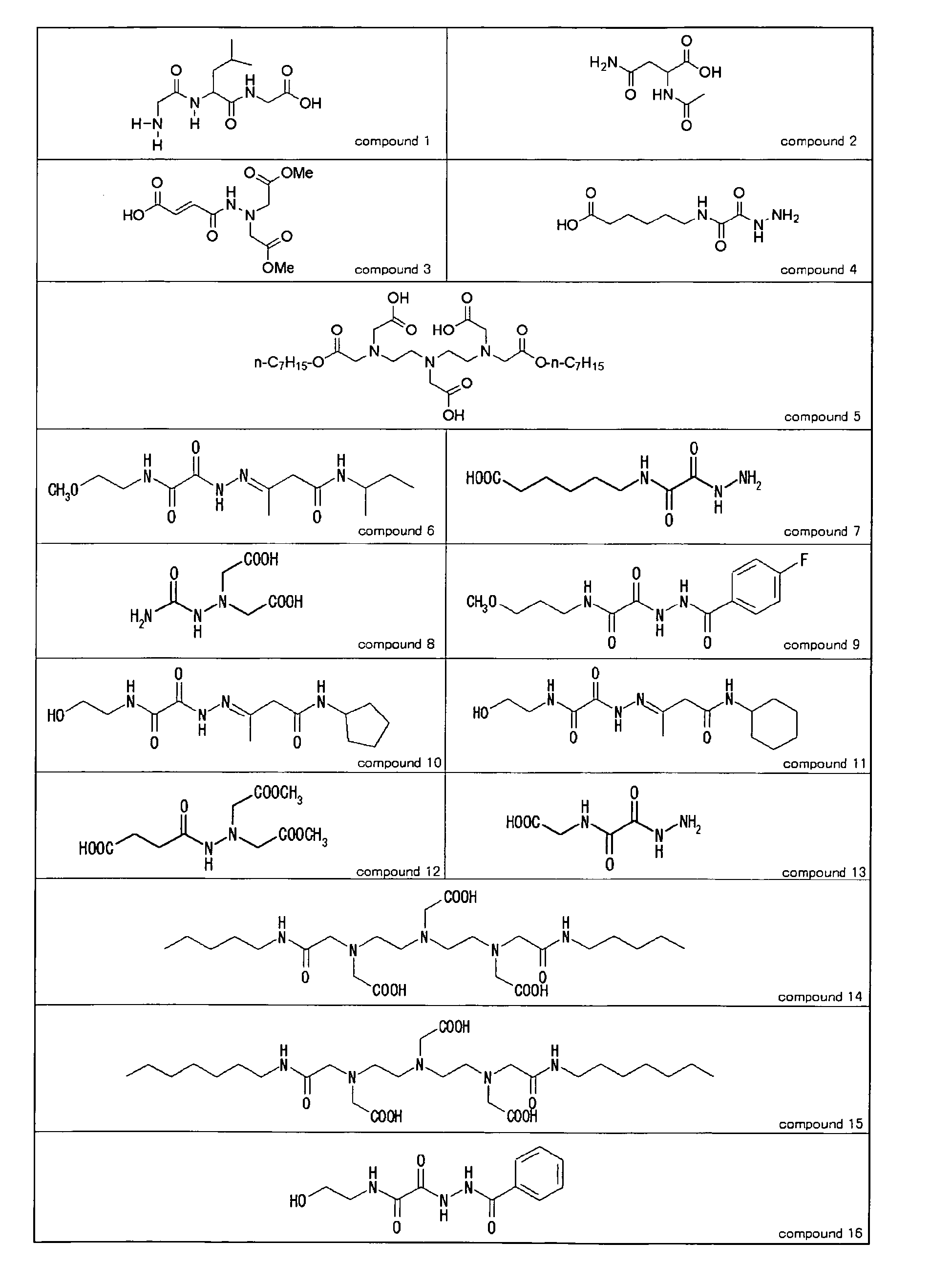

The phrase "wild type normal protein" herein includes protein comprising an amino acid sequence encoded by a normal gene, and protein comprising an amino acid sequence that is substantially identical to the amino acid sequence and having the same function as the protein comprising an amino acid sequence encoded by a normal gene. The protein, comprising an amino acid sequence that is substantially identical to the amino acid sequence encoded by a normal gene and having the same function as the protein comprising an amino acid sequence encoded by a normal gene, is not limited to full length protein as long as it has the function. More specifically, the protein comprising an amino acid sequence that is substantially identical to the amino acid sequence encoded by a normal gene means a protein that comprises an amino acid sequence having a homology with the amino acid sequence encoded by a normal gene of at least 80% or more, preferably 85% or more, more preferably 90% or more, further preferably 95% or more. For example, it means a protein that comprises an amino acid sequence having amino acid mutations such as substitution, deletion, insertion, and addition, or having modified amino acids in the amino acid sequence encoded by a normal gene. The number of mutated amino acids or modified amino acids is, for example, from 1 to 100, preferably from 1 to 30, more preferably from 1 to 20, even more preferably from 1 to 10, and still more preferably from 1 to several in number.

In the aforementioned aspect of the present invention, the method can be conducted in living bodies of mammals. In such a case, the compounds may be administered to the mammals. In one embodiment of the present invention, the mammals may be patients with genetic diseases caused by nonsense mutation, and the method may be conducted as a treatment method of genetic diseases caused by nonsense mutation.

The phrase "nonsense mutation mediated genetic disease" herein means a disease attributable to an internal termination codon in a gene (a premature termination codon) generated by such as a point mutation, deletion, and insertion in the gene which leads to inhibition of expression of protein having a normal function, or attributable to degradation of mRNA that contains the premature termination codon which leads to inhibition of protein expression. The genetic disease caused by nonsense mutation is not specifically limited, but is exemplified by the following: central nervous system diseases such as muscular dystrophy, Duchenne muscular dystrophy, multiple sclerosis, infantile neuronal ceroid lipofuscinosis, Alzheimer's disease, Tay-Sachs disease, neural tissue degeneration, and Parkinson's disease; autoimmune diseases such as chronic rheumatoid arthritis and graft-versus-host disease; inflammatory diseases such as arthritis; blood diseases such as hemophilia, von Willebrand disease, ataxia telangiectasia, thalassemia, familial erythrocytosis, and nephrolithiasis; collagen diseases such as osteogenesis imperfecta and cirrhosis; neurofibroma; bullous disease; lysosomal storage disease; Hurler's disease; familial cholesterolemia; cerebellar ataxia; tuberous sclerosis; immune deficiency; kidney disease; lung disease; cystic fibrosis; familial hypercholesterolemia; pigmentary retinopathy; amyloidosis; atheroscrerosis; gigantism; dwarfism; hypothyroidism; hyperthyroidism; aging; obesity; diabetes mellitus; Niemann-Pick disease; Marfan syndrome; and cancer. The term "cancer", such as cancer associated with a nonsense mutation of a suppressor gene such as p53 gene, includes all types of cancer, which is exemplified by lung cancer, colon and rectal cancer, stomach cancer, esophagus cancer, kidney cancer, pancreatic cancer, prostate cancer, breast cancer, uterus cancer, ovary cancer, skin cancer and brain tumor. A nonsense mutation mediated genetic disease is specifically exemplified by muscular dystrophy.

In the aforementioned aspect of the present invention, the compounds can be used with other compounds, or can be used as a composition of two or more compounds selected therefrom.

In another aspect of the present invention, a pharmaceutical composition for use in treatment or prevention of the genetic diseases caused by nonsense mutation is provided, wherein the pharmaceutical composition comprises as an effective ingredient a compound expressed by any one of the aforementioned formulas (I) to (V) or a pharmacologically acceptable salt thereof.

The pharmaceutical composition preferably comprises a compound expressed by any one of the aforementioned formulas (VI) to (X) or a pharmacologically acceptable salt thereof.

The pharmaceutical composition more preferably comprises a compound shown in the aforementioned table.

In the aforementioned aspect of the present invention, the pharmaceutical composition may contain a pharmacologically acceptable carrier or excipients. An amount of the compound used in the pharmaceutical composition is not limited as far as it is an effective amount for treatment. The genetic disease caused by nonsense mutation is not specifically limited, but is exemplified by the following: central nervous system diseases such as muscular dystrophy, Duchenne muscular dystrophy, multiple sclerosis, infantile neuronal ceroid lipofuscinosis, Alzheimer's disease, Tay-Sachs disease, neural tissue degeneration, and Parkinson's disease; autoimmune diseases such as chronic rheumatoid arthritis and graft-versus-host disease; inflammatory diseases such as arthritis; blood diseases such as hemophilia, von Willebrand disease, ataxia telangiectasia, thalassemia, familial erythrocytosis, and nephrolithiasis; collagen diseases such as osteogenesis imperfecta and cirrhosis; neurofibroma; bullous disease; lysosomal storage disease; Hurler's disease; familial cholesterolemia; cerebellar ataxia; tuberous sclerosis; immune deficiency; kidney disease; lung disease; cystic fibrosis; familial hypercholesterolemia; pigmentary retinopathy; amyloidosis; atheroscrerosis; gigantism; dwarfism; hypothyroidism; hyperthyroidism; aging; obesity; diabetes mellitus; Niemann-Pick disease; Marfan syndrome; and cancer. The term "cancer", such as cancer associated with a nonsense mutation of a suppressor gene such as p53 gene, includes all types of cancer, which is exemplified by lung cancer, colon and rectal cancer, stomach cancer, esophagus cancer, kidney cancer, pancreatic cancer, prostate cancer, breast cancer, uterus cancer, ovary cancer, skin cancer and brain tumor. A genetic disease caused by nonsense mutation is specifically exemplified by muscular dystrophy.

The pharmaceutical composition in the aspect of the present invention may contain, as active ingredients, the aforementioned compound and other compounds, or may contain a mixture of two or more aforementioned compounds.

The pharmacologically acceptable salt in the present specification is not specifically limited as far as it can be used in medicaments. Examples of a salt that the compound of the present invention forms with a base include the following: salts thereof with inorganic bases such as sodium, potassium, magnesium, calcium, and aluminium; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid: organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

Further, the compounds of the present invention include hydrates thereof, various pharmaceutically acceptable solvates thereof, and polymorphic crystals thereof.

The pharmaceutical compositions of the present invention can be formulated in various dosage forms, which are exemplified by the following: oral administration forms such as tablets, capsules, powders, granules, pills, liquids, emulsions, suspensions, solutions, spirits, syrups, extracts, and elixirs; parenteral administration forms such as injections, for example, subcutaneous injections, intravenous injections, intramuscular injections, and intraperitoneal injections; transdermal administration forms, plasters and pressure sensitive adhesives, ointments or lotions; intramouth administration forms such as sublingual forms and oral patch preparations; and nasal administration forms such as aerosols, but are not limited thereto. These preparations can be manufactured by using a known method generally used in a drug manufacturing process. In one embodiment of the present invention, the pharmaceutical composition of the present invention may be administered for treating muscular disease as an injection such as an intramuscular injection for administering directly into muscle.

The pharmaceutical compositions may contain various kind of ingredients generally used, for example, one or more pharmaceutically acceptable fillers, disintegrators, diluents, lubricants, flavoring agents, colorants, sweetening agents, corrigents, suspending agents, humectants, emulsifying agents, dispersing agents, auxiliary agents, preservatives, buffers, binders, stabilizers, and coating agents. In addition, the pharmaceutical composition of the present invention may be sustained-release dosage forms or extended-release dosage forms.

Dosage ranges of the pharmaceutical compositions are not particularly limited, and can be determined in accordance with the following: effectiveness of the ingredients contained therein; the administration form; the route of administration; the type of disease; the characteristics of the subject (e.g., body weight, age, symptomatic conditions, and whether a subject is taking other pharmaceutical agents); and the judgment of a physician in charge. In general, a suitable dosage may fall, for example, within a range of about 0.01 µg to 100 mg, per 1 kg of the body weight of the subject, and preferably within a range of about 0.1 µg to 1 mg, per 1 kg of body weight. However, the dosage may be altered using conventional experiments for optimization of a dosage that are well known in the art. The aforementioned dosage can be divided for administration once to several times a day. Alternatively, periodic administration once every few days or few weeks can be employed.

The pharmaceutical compositions may be administered to a patient whose biological sample obtained in advance is subjected to a study for presence or absence of premature termination codons in genes contained therein and is found to have a detected premature termination codon. A biological sample may be any ones insofar as it contains nucleic acids, and is exemplified by cells, bloods, cerebrospinal fluids, bronchoalveolar lavage fluids, expectorations, or other body fluids as well as biopsy tissues. Nucleic acid samples can be prepared from the biological samples for use. The nucleic acid samples can be prepared by well known nucleic acid preparation methods. The nucleic acid samples may be DNA or RNA. The nucleic acid samples prepared may be used directly for detection, or may be subjected to enzymatic amplification of predetermined region thereof by PCR or other amplification methods in advance for analysis. Detection of a termination codon can be carried out by using well known methods for detecting genetic mutations such as southern blot, polymerase chain reaction (PCR), short tandem repeat (STR), or restricted fragment length polymorphism. The detection method is not limited to the exemplified methods, and any method can be used insofar as it can detect a premature termination codon. Alternatively, the presence of a premature termination codon can be detected by measuring an amount of mRNA derived from the predetermined gene in the biological sample and detecting reduction of the amount of the mRNA compared to an amount of mRNA derived from the gene in a biological sample obtained from healthy subject. mRNA can be measures by using known analysis methods such as northern blotting.

In terms of a route of administration of the pharmaceutical composition, it may be either systemic administration or local administration. The route of administration that is appropriate for a particular disease, symptomatic condition, or other factors, should be selected. For example, parenteral administration including normal intravenous injection, intra-arterial administration, subcutaneous administration, intracutaneous administration, and intramuscular administration can be employed. Oral administration can be also employed. Further, transmucosal administration or transdermal administration can be employed.

Further, in another aspect of the present invention, a method of assay for a compound having read-through activity is provided, wherein the method comprises:
a) a step of preparing host cells or transgenic animals which are transfected with a vector comprising the following: i) promoter, ii) a first translation start codon and a first reporter gene located downstream of the promoter, iii) a second translation start codon and a second reporter gene located downstream of the first reporter gene, iv) a sequence being located between the first reporter gene and the second translation start codon, and comprising a premature termination codon derived from a responsible gene of a genetic disease caused by nonsense mutation, and v) a translation termination codon located downstream of the second reporter gene;
b) a step of administering a test compound to the host cells or the transgenic animals; and
c) a step of comparing a ratio of an expression amount of the second reporter gene to that of the first reporter gene between in the presence of the test compound and in the absence thereof.
The first reporter gene and the second reporter gene herein are selected from, for example, β -galactosidase gene, luciferase gene, green fluorescent protein gene, CAT(chloramphenicol acetyl transferase) gene, and the like. Preferably used are β-galactosidase gene and luciferase gene. In one embodiment of the present invention, use of β-galactosidase gene as the first reporter gene and luciferase gene as the second reporter gene is provided.

The term "read-through" herein means to skip over a premature termination codon in ribosomal translation, or to suppress degradation of mRNA that comprises a premature termination codon.

In the aforementioned aspect of the present invention, a sequence that comprises a premature termination codon derive from responsible genes for diseases caused by nonsense mutation is not specifically limited insofar as it is a sequence comprising a termination codon such as TAA, TAG, or TGA, in a reading flame. The sequence is preferably around 20 to 150 bp long. In one embodiment, the sequence maybe a sequence containing a sequence that comprises a premature termination codon of humans or animals having genetic disease caused by nonsense mutation including animal models for the diseases. For example, a gene that contains a premature termination codon in the dystrophin gene of mdx mice.

In terms of a promoter, CMV promoter, β-actin promoter, SV40 promoter, CMV/ β -actin hybrid promoter, and the like may be used. A promoter preferably used is CMV/ β -actin hybrid promoter.

The assay method according to the present invention may be conducted by using host cells or transgenic animals which are transfected with the aforementioned vector. In one embodiment of the aforementioned aspect of the present invention, the assay method may be conducted by using transgenic mice. Transfection of host cells or transgenic animals with the vector can be carried out by using known methods in the skilled person in the art of recombinant DNA technique.

In another aspect of the present invention, an agent for read-through of a premature termination codon generated by a nonsense mutation is provided, wherein the agent consists of a compound expressed by any one of the aforementioned formulas (I) to (V).

The phrase "an agent for read-through of a premature termination codon generated by a nonsense mutation" means an agent for causing or promoting read-through of the premature termination codon.

The agent for read-through may be exemplified by an agent for read-through comprising a compound expressed by any one of the aforementioned formulas (VI) to (X).

The agent for read-through may be preferably exemplified by a compound shown in the aforementioned table.

### Example 1

The following examples will explain the present invention, but are by no means intended to bring limited understanding on this invention.

### 1. reagents

Five compounds as shown below were obtained by purchase and were subjected to assays for their read-through activity.

Compound 1 was purchased from ChemBridge Corp. (CA, USA).

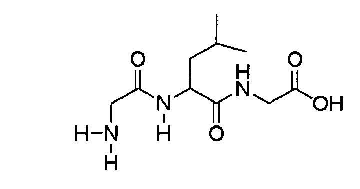

Compound 2 was purchased from Maybridge (Fisher scientific International Inc., England).

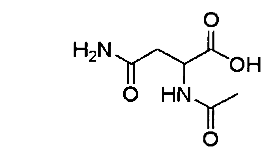

Compound 3 was purchased from Nanosyn Inc. (CA, USA).

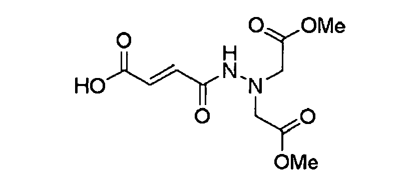

Compound 4 was purchased from Nanosyn Inc. (CA, USA).

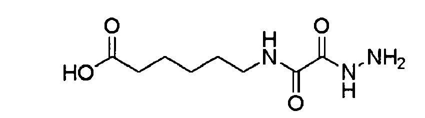

Compound 5 was purchased from Nanosyn Inc. (CA, USA).

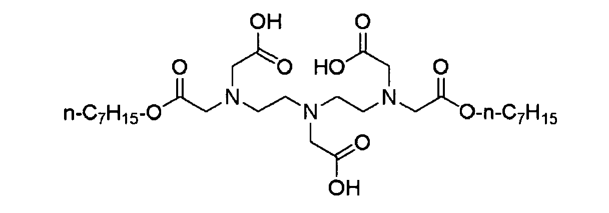

### 2. Construction of assay system

### 1) Construction of expression plasmid

### a) Modification of luciferase gene

In order to make luciferase gene linked to β-galactosidase gene, the following primer was prepared to generate a restriction enzyme site (Pst I) in front of the start codon for luciferase.

Forward primer: 5'-cccAAGCTTCTGCAG-atggaagacgccaaaaacataaag-3' (SEQ ID NO: 1)
Reverse primer: 5' -cccTTCGAA-gtactcagcgtaagtgatgtccacc-3' (SEQ ID NO: 2)

Cloning of PCR fragments was conducted by using plasmid DNA that contains luciferase gene, named pGL3-Basic Vector (Promega, USA), and by utilizing Hind III site and Nsp V site of the vector. PCR was carried out under a condition of 35 cycles consisting of annealing temperature of 60 °C and elongation time of 30 sec. The DNA polymerase used was KOD Plus DNA Polymerase (TOYOBO). As a result, an amplified DNA with a predetermined length of approximately 0.17kb was detected.

The PCT products thus obtained was cleaved with Hind III/Nsp V followed by ligation into Hind III/Nsp V site of pGL2-Basic Vector. The obtained vector was used for transfection of cells of E. coli JM109 which were then subjected for extraction of plasmid DNA. The obtained plasmid DNAs were cleaved with Hind III/Nsp V, and plasmids that were detected to have a DNA fragment with predetermined length were selected. The selected plasmids were subjected for a DNA sequence analysis to confirm the introduced predetermined modification in the sequence.

### b) modification of galactosidase gene

In order to remove the termination codon from β-galactosidase gene and insert a nonsense sequence containing a termination codon of TAA and to add a Pst I site thereto for ligation to luciferase gene, oligo DNAs having the following sequence and the complementary sequence thereof were synthesized for use as a primer.

5 ' -GAATTC-CAGCTGAGCGCCGGTCGCTACCATTACCAGTTGGTCTGGTGT
CAAAAA-AAAGAGCAATAAAATGGC-CTGCAG-3' (SEQ ID NO: 3)

The aforementioned sequences were annealed and ligated into β-galactosidase Control Vector with approximately 2.7kb (Promega, USA) at the EcoRI/Pst I site. The obtained vector was used for transformation of cells of E. coli JM109 which were then subjected for extraction of plasmid DNA. The obtained plasmid DNAs were cleaved with Eco RI/Pst I, and plasmids that were detected to have a DNA fragment with predetermined length were selected. The selected plasmids were subjected for a DNA sequence analysis to confirm the sequence into which the synthetic DNA fragment was inserted between the 6814^{th} Eco RI and the 4169^{th} Pst I of β -galactosidase gene.

The confirmed plasmid was cleaved with Eco RI, ligated into β-galactosidase Control Vector with approximately 3.7kb, and used for transformation of cells of E. coli JM109 which were then subjected for extraction of plasmid DNA. The obtained plasmid DNAs were cleaved with Hind III/Pst I, and plasmids in which ligation was conducted in a predetermined directional manner were selected.

### c) ligation of the modified β-galactosidase gene and the modified luciferase gene

The plasmid containing the modified β-galactosidase gene that was prepared in the aforementioned step b) was ligated to the plasmid containing the modified luciferase gene that was prepared in the aforementioned step a) at the Hind III/Pst I site, and then used for transformation of cells of E. coli JM109 which were then subjected for extraction of plasmid DNA. The obtained plasmid DNAs were cleaved with Hind III/Pst IBam HI, and plasmids that were detected to have a DNA fragment with a desired length were selected.

### d) ligation into pCAGGS

The plasmid obtained in the step c) was cleaved with Hind III/Xba I followed by subjecting to blunting treatment. The resultant DNA fragment was ligated into a plasmid DNA that was obtained by cleaving pCAGGS (Oriental Yeast Co., Ltd.) with Eco RI followed by subjecting to blunting treatment and dephosphorylation treatment. The plasmid was then used for transformation of cells of E. coli DH5α which were then subjected to extraction of plasmid DNA. The obtained plasmid DNAs were cleaved with Sal I/Eco RI, and plasmids that were detected to have a DNA fragment with a predetermined length of 1.8kb, 3.0kb, or 5.0kb were selected. The selected plasmids were subjected to DNA sequence analysis for blunting region to confirm the predetermined base sequence.

### e) Construction of expression plasmid containing a termination codon of TGA or TAG

The expression plasmid containing a termination codon TGA and the expression plasmid containing a termination codon TAG were constructed using SEQ ID NO: 4 and SEQ ID NO: 5 respectively, in a same manner as described in the aforementioned steps a) to d) by using oligonucleotides having the following sequence and the complementary sequence instead of using the oligo DNA (SEQ ID NO: 3) used in the modification of β-galactosidase gene.

5 ' -GAATTC-CAGCTGAGCGCCGGTCGCTACCATTACCAGTTGGTCTGGTGT
CAAAAA-AAAGAGCAATGAAATGGC-CTGCAG-3' (SEQ ID NO: 4)
5'-GAATTC-CAGCTGAGCGCCGGTCGCTACCATTACCAGTTGGTCTGGTGTCAAAAA-
AAAGAGCAATAGAATGGC-CTGCAG-3' (SEQ ID NO: 5)

### 2) Production of transgenic mice

Production of transgenic mice was conducted based on the method reported by Gordon et al (Proc. Natl. Acad. Sci., Vol. 77, p. 7380-7384). C57BL/6J JCL female mice were induced to superovulate and mated with mice of the same strain for obtaining eggs. After that, the obtained fertilized eggs (pronucleous stage embryos) were subjected to microinjection into the male pronucleus with the prepared DNA fragment which termination codon was TAA. The fertilized eggs were then transplanted into oviducts of pseudofertilized CD-1 ICR female mice. The mice were allowed for natural delivery.

### 3. assay for read-through activity

The aforementioned compounds 1 to 5 in saline solutions (Otsuka Pharmaceutical Co., Ltd., 100µg/100µL) were injected subcutaneously into abdominal region of the transgenic mice produced by the aforementioned method each 24 hours for 7 days. The mice were euthanized on day 8 by inhalation of overdose ether followed by excision of femoral and tibial skeletal muscles. The muscles were minced with ophthalmic scissors and added with Reporter Lysis Buffer (Promega, USA) with a three times weight of wet weight of the muscles, followed by homogenized of tissues using physcotron (Microtec Co., Ltd.) together with glass strips. After freeze-drying, a centrifuged supernatant was transferred into 96 well plates and subjected to measurements of β-galactosidase activity by using Beta-Glo Assay system (Promega, USA) and of luciferase activity by using Bright-Glo Assay System (Promega, USA). The measurements were conducted with luminometer (ATTO). Luciferase activity was standardized by dividing a luciferase activity value by a β-galactosidase activity value and multiplying with 10⁴, and the resultant value was displayed as a read-through efficiency. The results were shown in Figure 1.

Each of the groups administered with the compounds 1 to 5 was found to have a significantly higher read-through activity than control saline.

### Example 2

The aforementioned compound 2 was studied for its effect of administration on a muscular dystrophy mouse model. An mdx mouse was used as a muscular dystrophy mouse model.

The compound 2 was administered forcibly into the stomach of an mdx mouse at a dose of 4 or 400mg/kg/day for 7 days, and then biochemical and immuno-histochemical analyses were conducted. The results showed reduced serum creatine kinase activity and accumulated dystrophin in muscles of, for example, the hind limb. The group administered with the compound 2 showed favorable results compared to a positive control group administered with gentamicin.

### Example 3

The aforementioned compound 2 was studied for side effects in mice.

The compound 2 was injected subcutaneously to an mdx mouse at a dose of 4 or 400mg/kg/day for 14 days. There was no decrease in body weight during the administration period, and no abnormal values were observed in a hearing test that measures auditory brainstem response and in eighteen serological and biochemical tests such as for total protein, albumin, aspartate aminotransferase, alanine aminotransferase, alkaline phosphatase, γ-glutamyl transpeptidase, creatinine, glucose, triglyceride, phospholipid, total cholesterol, sodium, potassium, chlorine, calcium, inorganic phosphorus, total bilirubin, and albumin/glucose ratio.

### SEQUENCE LISTING FREE TEXT

SEQ ID NO: 1: designed oligo DNA for use as a primer
SEQ ID NO: 2: designed oligo DNA for use as a primer
SEQ ID NO: 3: designed oligo DNA for use as a primer
SEQ ID NO: 4: designed oligo DNA for use as a primer
SEQ ID NO: 5: designed oligo DNA for use as a primer

## Claims

1. A method of producing wild type normal protein from a gene with a premature termination codon being generated by a mutation, wherein the method comprises conducting ribosomal translation in the presence of a compound expressed by any one of the following formulas (I) to (V) or a pharmacologically acceptable salt thereof : 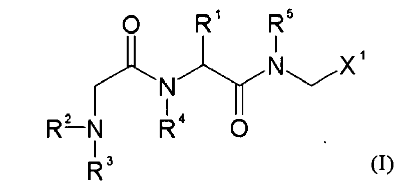 wherein, in Formula (I), R¹ represents C₁₋₁₀ alkyl; R² and R³ are independently selected from hydrogen atom and C₁₋₁₀ alkyl which may have a substituent; R⁴ and R⁵ are independently selected from hydrogen atom and C₁₋₁₀ alkyl; X¹ represents carboxy, lower alkoxycarbonyl which may have a substituent, carbamoyl which may have a substituent, or hydrazinocarbonyl which may have a substituent, 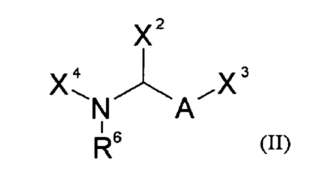 wherein, in Formula (II), X², X³ and X⁴ independently represent carboxy, alkoxycarbonyl which may have a substituent, carbamoyl which may have a substituent, or hydrazinocarbonyl which may have a substituent; R⁶ represents hydrogen atom or C₁₋₁₀ alkyl which may have a substituent; A represents alkylene which may have a substituent, 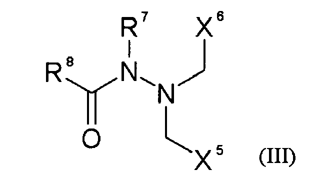 wherein X⁵ and X⁶ are independently selected from carboxy, alkoxycarbonyl which may have a substituent, and carbamoyl which may have a substituent; R⁷ represents hydrogen atom or C₁₋₁₀ alkyl which may have a substituent; R⁸ represents amino which may have a substituent, C₁₋₁₀ alkyl which may have a substituent, or C₁₋₁₀ alkenyl which may have a substituent, 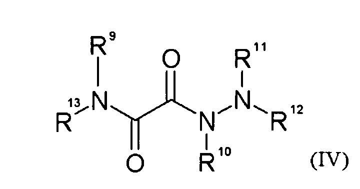 wherein R⁹, R¹⁰ and R¹³ independently represent hydrogen atom or C₁₋₁₀ alkyl which may have a substituent; R¹¹ and R¹² independently represent hydrogen atom, C₁₋₁₀ alkyl which may have a substituent, or carbonyl which is substituted with an aromatic substituent, or R¹¹ and R¹² may mutually bonded to form double bond leading to =CR¹⁷R¹⁸, wherein R¹⁷ and R¹⁸ are independently selected from hydrogen atom, C₁₋₁₀ alkyl, and carbamoylalkyl which may have a substituent, 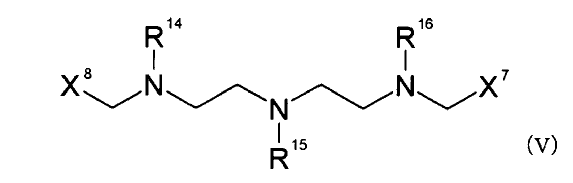 wherein X⁷ and X⁸ are independently selected from carboxy, alkoxycarbonyl which may have a substituent, and carbamoyl which may have a substituent; R¹⁴, R¹⁵ and R¹⁶ independently represent hydrogen atom or C₁₋₁₀ alkyl which may have a substituent.

2. The method according to claim 1, wherein the method comprises administering the compound to a mammal, and wild type normal protein is produced in a living body of the mammal.

3. The method according to claim 2, wherein the mammal is a patient with a genetic disease caused by nonsense mutation.

4. The method according to claim 3, wherein the genetic disease caused by nonsense mutation is selected from a group consisting of muscular dystrophy, Duchenne muscular dystrophy, multiple sclerosis, infantile neuronal ceroid lipofuscinosis, Alzheimer's disease, Tay-Sachs disease, neural tissue degeneration, Parkinson's disease, chronic rheumatoid arthritis, graft-versus-host disease, arthritis, hemophilia, von Willebrand disease, ataxia telangiectasia, thalassemia, nephrolithiasis, osteogenesis imperfecta, cirrhosis, neurofibroma, bullous disease, lysosomal storage disease, Hurler's disease, familial cholesterolemia, cerebellar ataxia, tuberous sclerosis, familial erythrocytosis, immune deficiency, kidney disease, lung disease, cystic fibrosis, familial hypercholesterolemia, pigmentary retinopathy, amyloidosis, atheroscrerosis, gigantism, dwarfism, hypothyroidism, hyperthyroidism, aging, obesity, diabetes mellitus, Niemann-Pick disease, Marfan syndrome, and cancer.

5. The method according to claim 3, wherein the genetic disease caused by nonsense mutation is muscular dystrophy.

6. A pharmaceutical composition for use in treatment or prevention of a genetic disease caused by nonsense mutation, wherein the pharmaceutical composition comprises as an effective ingredient a compound expressed by any one of the formulas (I) to (V) described in claim 1 or a pharmacologically acceptable salt thereof.

7. The pharmaceutical composition according to claim 6, wherein the genetic disease is selected from a group consisting of muscular dystrophy, Duchenne muscular dystrophy, multiple sclerosis, infantile neuronal ceroid lipofuscinosis, Alzheimer's disease, Tay-Sachs disease, neural tissue degeneration, Parkinson's disease, chronic rheumatoid arthritis, graft-versus-host disease, arthritis, hemophilia, von Willebrand disease, ataxia telangiectasia, thalassemia, nephrolithiasis, osteogenesis imperfecta, cirrhosis, neurofibroma, bullous disease, lysosomal storage disease, Hurler's disease, familial cholesterolemia, cerebellar ataxia, tuberous sclerosis, familial erythrocytosis, immune deficiency, kidney disease, lung disease, cystic fibrosis, familial hypercholesterolemia, pigmentary retinopathy, amyloidosis, atheroscrerosis, gigantism, dwarfism, hypothyroidism, hyperthyroidism, aging, obesity, diabetes mellitus, Niemann-Pick disease, Marfan syndrome, and cancer.

8. The pharmaceutical composition according to claim 6, wherein the genetic disease is muscular dystrophy.

9. A method of assay for a compound having read-through activity, wherein the method comprises:
a) a step of preparing host cells or transgenic animals which are transfected with a vector comprising the following: i) promoter, ii) a first translation start codon and a first reporter gene located downstream of the promoter, iii) a second translation start codon and a second reporter gene located downstream of the first reporter gene, iv) a sequence being located between the first reporter gene and the second translation start codon, and comprising a premature termination codon derived from a responsible gene of a genetic disease caused by nonsense mutation, and v) a translation termination codon located downstream of the second reporter gene;
b) a step of administering a test compound to the host cells or the transgenic animals; and
c) a step of comparing a ratio of an expression amount of the second reporter gene to that of the first reporter gene between in the presence of the test compound and in the absence thereof.

10. An agent for read-through of a premature termination codon generated by a nonsense mutation, wherein the agent consists of a compound expressed by any one of the formulas (I) to (V) described in claim 1, a hydrate thereof, a solvate thereof, or a salt of any of the foregoing.

11. A method of producing wild type normal protein from a gene with a premature termination codon being generated by a mutation, wherein the method comprises conducting ribosomal translation in the presence of a compound expressed by any one of the following formulas (VI) to (X) or a pharmacologically acceptable salt thereof: 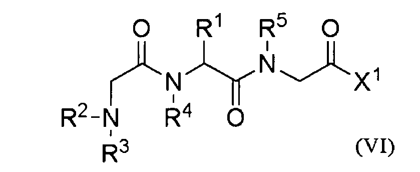 wherein, in the formula, R¹ represents C₁₋₁₀ alkyl; R² and R³ are independently selected from hydrogen atom, C₁₋₁₀ alkyl, and C₁₋₁₀ alkylcarbonyl; R⁴ and R⁵ are independently selected from hydrogen atom and C₁₋₁₀ alkyl; X¹ represents hydroxy, amino, C₁₋₁₀ alkoxy, C₁₋₁₀ alkylamino or di(C₁₋₁₀ alkyl)amino, 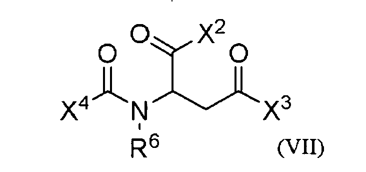 wherein, in the formula, X², X³ and X⁴ are independently selected from hydroxy, amino, C₁₋₁₀ alkoxy, C₁₋₁₀ alkylamino, and di(C₁₋₁₀ alkyl)amino; R⁶ represents hydrogen atom or C₁₋₁₀ alkyl, 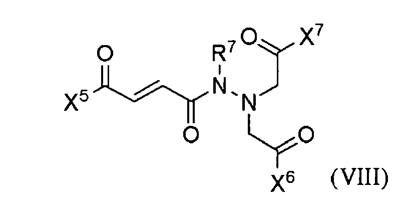 wherein X⁵, X⁶ and X⁷ are independently selected from hydroxy, amino, C₁₋₁₀ alkoxy, C₁₋₁₀ alkylamino, and di(C₁₋₁₀ alkyl)amino; R⁷ represents hydrogen atom or C₁₋₁₀ alkyl, 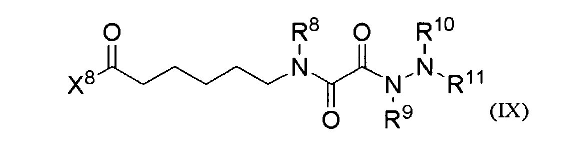 wherein X⁸ represents hydroxy, amino, C₁₋₁₀ alkoxy, C₁₋₁₀ alkylamino, and di(C₁₋₁₀ alkyl)amino; R⁸ and R⁹ independently represent hydrogen atom or C₁₋₁₀ alkyl; R¹⁰ and R¹¹ are independently selected from hydrogen atom, C₁₋₁₀ alkyl and C₁₋₁₀ alkylcarbonyl, 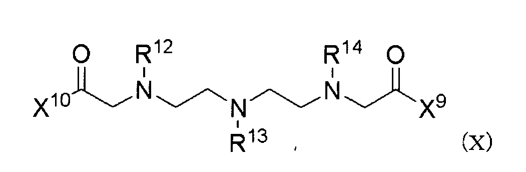 wherein X⁹ and X ¹⁰ are independently selected from hydroxy, amino, C₁₋₁₀ alkoxy, C₁₋₁₀ alkylamino, and di(C₁₋₁₀ alkyl)amino; R¹², R¹³ and R¹⁴ are independently selected from hydrogen atom, C₁₋₁₀ alkyl, C₁₋₁₀ alkylcarbonyl and -CH₂COX¹¹; X¹¹ represents hydroxyl, amino, C₁₋₁₀ alkoxy, C₁₋₁₀ alkylamino, and di(C₁₋₁₀ alkyl)amino.

12. A pharmaceutical composition for use in treatment or prevention of genetic diseases caused by nonsense mutation, wherein the pharmaceutical composition comprises as an effective ingredient a compound expressed by any one of the formulas (VI) to (X) described in claim 11 or a pharmacologically acceptable salt thereof.


SEQUENCE LISTING

<110> THE UNIVERSITY OF TOKYO
UNIVERSITY OF MARYLAND, BALTIMORE
MATSUDA, Ryoichi
SHIOZUKA, Masataka
MACKERELL, JR, Alexander

<120> METHOD FOR TREATING DISEASE DUE TO NONSENSE MUTATION

<130> GP07-1009PCT

<150> US 60/806,580
<151> 2006-07-05

<160> 5

<170> PatentIn version 3.1

<210> 1
<211> 39
<212> DNA
<213> artificial

<220>
<223> Designed oligoDNA for use as a primer

<400> 1
cccaagcttc tgcagatgga agacgccaaa aacataaag 39

<210> 2
<211> 34
<212> DNA
<213> artificial

<220>
<223> Designed oligoDNA for use as a primer

<400> 2
cccttcgaag tactcagcgt aagtgatgtc cacc 34

<210> 3
<211> 78
<212> DNA
<213> artificial

<220>
<223> Designed oligoDNA for use as a primer

<400> 3
gaattccagc tgagcgccgg tcgctaccat taccagttgg tctggtgtca aaaaaaagag 60

caataaaatg gcctgcag 78

<210> 4
<211> 78
<212> DNA
<213> artificial

<220>
<223> Designed oligoDNA for use as a primer

<400> 4
gaattccagc tgagcgccgg tcgctaccat taccagttgg tctggtgtca aaaaaaagag 60

caatgaaatg gcctgcag 78

<210> 5
<211> 78
<212> DNA
<213> artificial

<220>
<223>    Designed oligoDNA for use as a primer

<400>    5
gaattccagc tgagcgccgg tcgctaccat taccagttgg tctggtgtca aaaaaaagag    60

caatagaatg gcctgcag    78